# NEW EUROPEAN PATENT SPECIFICATION

(11) **EP 0 533 408 B2**
(45) Date of publication and mention of the opposition decision: **28.02.2001**
(45) Mention of the grant of the patent: 27.12.1996
(21) Application number: 92308279.6
(22) Date of filing: 11.09.1992
(51) Int. Cl.: A61K 7/48, A61K 7/06, A61K 38/00

(54) **Cosmetics and pharmaceuticals containing extensins**
Kosmetika und Pharmazeutika enthaltend Extensin
Compositions cosmétiques et pharmaceutiques contenant de l'extensine

(30) Priority: 18.09.1991 US 761574
(43) Date of publication of application: 24.03.1993
(73) Proprietor: Revlon Consumer Products Corporation, New York, NY 10022 (US)
(72) Inventor: Wolf, Barbara, Scarsdale, New York 10583 (US); Tietjen, Marlene, New York, New York 10011 (US)
(74) Representative: Sanderson, Laurence Andrew

(56) References cited:
- S.T.N., FILE SUPPLIER, KARLSRUHE, DE, File Chemical Abstracts, vol. 111, n 201338a
- S.T.N., FILE SUPPLIER, KARLSRUHE, DE, File Chemical Abstracts, vol 116, n 200854g
- P. Marcet, Cosmetic News 75, November/December 1990, p. 410-412
- J.J. Smith et al, Phytochemistry, vol. 23, n° 6, 1984, p. 1233-1239
- J.B. Cooper and J.E. Varner, plant Physiol. vol. 76, 1984, p. 414-417
- J.W. Heckman, Plant Physiol, vol. 86, 1988, p. 848-856
- G. Proserpio and G. Scarlatini, Cosmetic News, vol. 60, May/June 1988, p. 169-174
- Phytochemistry, 1986, vol. 25, n° 5, p. 1021-1030
- Seifen, Öle, Fette, Wachse Journal, vol. 117, n° 18, p. 717-721
- Cosmetic News XV, 82/92, G. Scarlatini "Estensina - Proteina vegetale colageno-simile"

## Description

The invention is in the field of cosmetic and pharmaceutical compositions containing extensin proteins.

### Background of the Invention

Cosmetics companies are particularly interested in formulating treatment cosmetics which exert beneficial effects on skin and hair. Since it is known that various animal derived substances provide beneficial effects to skin and hair, it has become popular to incorporate these substances into cosmetics and personal care products. For example, animal collagen is known to have moisturizing and film forming properties, and is a popular additive to treatment cosmetics. Animal collagen protein is the main component of connective tissues, skin, muscles and tendons. It is a fibrous protein of about 100,000 molecular weight, rich in proline and hydroxyproline, and structurally analogous to a three-stranded rope in which each strand is a polypeptide chain. Collagen is responsible for most of skin structure. In the course of aging the polypeptide chains of collagen polymerize. The result is "cross linking", which causes wrinkling of the skin as well as reduction in skin elasticity.

Other non-collagen animal proteins such as plasma proteins, placental proteins, or proteins from milk sources are also popular as cosmetics additives, as well as proteins from lower animals such as silkworm, fish, bacteria, yeast, or non-specified marine sources.

Extensins are a family of plant derived hydroxyproline rich glycoproteins (HRGP) firmly bound to the primary cell wall of several species of monocotyledonous and dicotyledenous plants. Extensins are also rich in serine, valine, tyrosine, lysine, and in some instances threonine, and the polypeptide' backbone comprises repeating hydroxyproline units in conjunction with other basic amino acids such as valine, lysine, proline, tyrosine, histidine, serine, and threonine. The hydroxyproline component is heavily glycosylated. Extensins play a role in growth, regulation, stress response, cell-cell recognition, and reproductive physiology of plants. The protein is widely distributed throughout the plant kingdom. Extensins are generally insoluble in muro because of their extensive cross linking, so the intact protein has not been isolated from mature plants, which has made the scientific study of intact extensin protein much more difficult. A hydrolyzed form of extensin is available commercially through Centerchem, Stamford, Connecticut. However, this hydrolyzed product contains very small polypeptide chains having molecular weights of 100-1500 daltons. The estimated size of intact extensin protein is greater than 100,000 daltons, or about 100,000-150,000 daltons.

It is known that prior to incorporation of insoluble cross linked extensin into the plant cell wall it exists in a soluble form which can be isolated by salt extraction of cell suspension cultures containing the soluble precursor form.

It has unexpectedly been discovered that this soluble form of extensin may be incorporated into cosmetics as an analog for animal collagen and will act to smooth, tighten, and enhance skin texture. Extensins also contain large amounts of humectant sugars which are capable of binding water, thus making them ideal humectants.

### Summary of the Invention

The invention is directed to cosmetic compositions comprising cosmetically effective film-forming concentrations of a soluble precursor-form extensin protein as defined in claim 1.

The invention is directed to pharmaceutical compositions comprising pharmaceutically effective film-forming concentrations of a soluble precursor-form extensin protein as defined in claim 1.

The invention is directed to the use of a soluble precursor-form of extensin protein as defined in claim 1 in the manufacture of a cosmetic or pharmaceutical composition for applying to the skin as a method of moisturizing and forming a film on skin.

### Description of Drawings

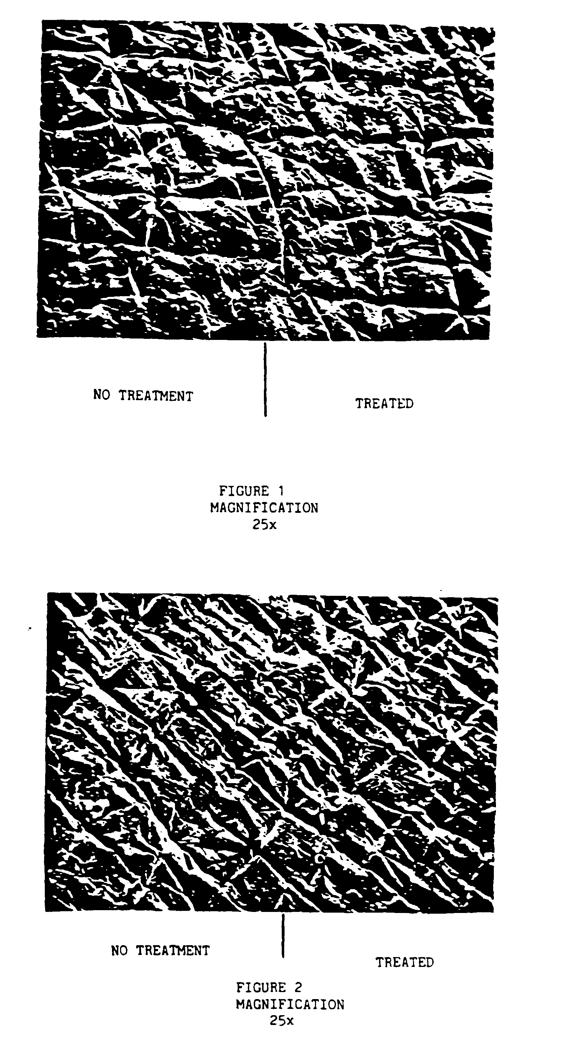

Figure 1 shows a scanning electron micrograph of a silicone replica of skin at 25X magnification. About 40 microliters of a 0.2% solution of extensin from carrots was applied to the right half of an area 15 millimeters in diameter (about 44 square millimeters). The left half of the area was left untreated.

Figure 2 shows a scanning electron micrograph of a silicone replica of skin at 25X magnification. About 40 microliters of a 0.2% solution of a high molecular weight non-extensin protein from wheat was applied to the right half of an area 15 millimeters in diameter (44 square millimeters of treated surface area), while the left half was left untreated. A comparison of both figures reveals that skin treated with extensin protein has an appreciably more intact and visible film than skin treated with high molecular weight non-extensin wheat protein or untreated skin.

### Detailed Description

The term "extensin" or "extensin protein" means a soluble hydroxyproline-rich glycoprotein analogue to animal collagen which is extracted from plant cell walls. Structurally, extensins are comprised of a polypeptide backbone comprising repeating hydroxyproline units in conjunction with other basic amino acids such as valine, lysine, proline, tyrosine, histidine, serine, and threonine. The term "extensin" includes the intact extensin protein which has a molecular weight of about 100,000 daltons up to 150,000 daltons.

Extensin hydrolysates may be purchased from Centerchem, Stamford Connecticut, and exhibit molecular weights of 100-1500 daltons.

The intact extensin proteins used in the cosmetic compositions of the invention are described in the following publications and may be extracted from various plants by methods described in these publications: Biochimica Et Biophysica Acta. Vol. 257(1972) 421-432; Plant Physiology, Vol. 76 (1984) 414-417; Phylochemistry, Vol. 23, No. 6 (1984) 1233-1239; Plant Physiology Vol. 85 (1987) 823-827; Plant Physiology, Vol. 87 (1987) 616-621; Plant Physiology. Vol. 86 (1988) 848-856; Phytochemistry, Vol. 25. No. 5 (1986) 1021-1030; all of which are hereby incorporated by reference.

Generally, soluble extensin precursors may be extracted from plant species in the growth phase by salt extraction of crude extensin protein fractions from concentrated plant cell suspension pellets or explant homogenates. Various metallic salt solutions are suitable for this purpose including aluminum chloride, calcium chloride, lapyrium chloride, sodium chloride, etc. The crude extensin protein extracts may then be further purified by acid precipitation of contaminants using acids such as trichloroacetic acid. Extensin proteins are generally soluble in 5-10% trichloroacetic acid. Then standard protein purification methods may be used for further purification, for example size exclusion affinity chromatography or ion exchange chromatography.

A 0.5% solution of extensin generally produces a flexible film that keeps the skin pliable and firm for five to six hours. The cosmetic benefits are seen in cosmetic and pharmaceutical compositions as outlined below.

The extensin extracts may be incorporated into a variety of cosmetic and pharmaceutical compositions. Cosmetic or pharmaceutical compositions containing effective amounts of extensins improve texture, smoothness, and moisture content of the skin.

The term "cosmetic composition" means a composition applied externally to skin, nails, or hair of the human body, for purposes of beautifying, colouring, conditioning, or protecting the bodily surface. Examples of cosmetic compositions in accordance with the invention include lotions, creams, moisturizers, gels, sun screens, makeup, cleansers, shampoos, hair conditioners, skin firming compositions, protein concentrates, after shaves, eyeshadows, blushes and nail enamels.

The term "pharmaceutical composition" means a composition applied externally to the skin, hair, or nails of the human body for therapeutic purposes. Examples of pharmaceutical compositions in accordance with the invention include ointments, creams, lotions, gels and solutions.

The invention comprises cosmetic compositions comprising a cosmetically effective amount of extensin protein. A cosmetically effective amount of extensin protein in accordance with the invention is 0.01-30% by weight of the total composition, with 0.1-10% preferred, and 0.5-5% most preferred.

The extensin proteins may be incorporated into a wide variety of cosmetic compositions. For example, cosmetically effective amounts of extensin may be incorporated into moisturizing lotions for application to human facial or body skin. These lotions generally contain from about 20-80% oil and 10-80% water in an emulsion form. In addition the moisturizing lotion may contain humectants, emollients, surfactants, fragrances and preservatives. About 5-10% humectant, about 5-20% emollient, and about 0.5-10% surfactant are suggested.

Extensins may be easily incorporated into moisturizing creams. Creams generally contain from about 20-70% water and about 30-70% oil. In addition, creams may contain a variety of humectants, emollients, surfactants, preservatives, and fragrances. About 5-10% humectant, about 5-20% emollient, and about 0.5-10% surfactant are suggested.

Extensins may also be incorporated into treatment makeups. Generally makeup formulations comprising 5-70% oil, 10-95% water, and about 5-40% pigment are suitable. In addition the makeup may contain surfactants, silicone, humectants, emollients, preservatives, fragrances, etc. Generally 0.5-10% surfactant, 0.1-30% silicone, 5-10% humectant, 0.1-30% emollient, and 0.1-5% preservative is suggested.

Extensins may also be incorporated into colored cosmetics such as eyeshadow or blush. For example, a suitable eyeshadow comprises 5-40% pigments, 1-50% oil, and 1-20% waxes. Additionally the composition may contain one or more of 10-60% water, 0.5-30% surfactant, 1-10% humectants, 0.1-5% preservative, and 0.1-20% silicone.

Extensins are also suitable for incorporation into shampoos and hair conditioners. Suitable shampoo formulations include 1-40% surfactant and 10-90% water. Suitable hair conditioning formulations include 30-95% water, 0.5-30% conditioning ingredients such as emollients, proteins, shine enhancers, and so forth, and 1-40% surfactant. Hair conditioners and shampoos may also contain thickeners and silicone. About 0.05-15% silicone is suggested in shampoos and hair conditioners.

Extensins may also be incorporated into cleansers, aftershaves, toners, fragrance splashes, and even nail treatment products or nail enamels. For example, fragrance splashes, aftershaves and toners, generally comprise about 10-70% alcohol. In addition, 0.01-5% surfactant may be added as well as 1-5% humectants, and up to 25% perfume.

Extensins may be incorporated into traditional nail enamels or nail treatment products which generally comprise about 1-40% film former, 10-50% resin, and 10-70% solvent, in addition to the usual plasticizers, pigments, and wetting agents.

The invention is also directed to pharmaceutical compositions comprising pharmaceutically effective amounts of extensin protein.

A pharmaceutically effective amount in accordance with the invention means about 0.01-30% of extensin protein, with 0.1-10% preferred, and 0.5-5% most preferred. The extensin proteins may be incorporated into suitable pharmaceutical vehicles such as lotions, creams, ointments, gels, or solutions. Suitable ointments are hydrophilic ointments (USP) or petrolatum and cosmetically effective amounts of extensin, protein are incorporated into the ointment for topical application to skin. Suitable lotions and creams are are as mentioned previously for cosmetic compositions. Solutions are made by mixing solutions of extensin protein in deionized water for application to human skin. Gels are made by mixing 1-90% water with a suitable polymer.

Suitable humectants for use in the cosmetic compositions of the invention include glycerin, propylene glycol, butylene glycol, urea, sorbitol, sodium PCA, gelatin, polyethylene glycols, sodium lactate, hyaluronic acid, and so on.

Suitable ernollients include glyceryl stearate, cetyl alcohol, stearyl alcohol, isopropyl stearate, stearyl alcohol, stearyl stearate, isopropyl stearate, stearic acid, isobutyl palmitate, isocetyl stearate, oleyl alcohol, sebacates, myristates, palmitates, squalenes, glyceryl monooleate, oleic acids, lanolin, acetylated lanolin alcohols, petrolatum, mineral oils, palmitic acids, isostearyl neopentanoate, etc.

A variety of surfactants may be used in the compositions of the invention including amphoteric, anionic, cationic or nonionic surfactants. Suitable amphoteric surfactants indude imidazolines, betaines, and amino acid salts. Suitable anionic surfactants include fatty acid soaps, salts of higher alkyl sulfates, n-acyl sarcosinates, salts of phosphates, sulfosuccinate salts, alkyl benzene sulfonates, salts of N-acyl glutamate, polyoxyethylene alkyl ether carboxylic acids, and so on. Cationic surfactants include alkyl trimethyl ammonium salts, alkyl pyridinium salts, alkyl quaternary ammonium salts, polyamine fatty acid derivatives, etc. Nonionic surfactants include lipophilics such as sorbitan fatty acid esters, glycerol fatty acids, propylene glycol fatty acid esters; hydrophilics such as polyoxyethylene sorbitan fatty acid esters, polyoxyethylene glycerol fatty acid esters, polyoxyethylene fatty acid esters, polyoxyethylene alkyl ethers, pluronics, polyoxyethylene alkyl phenyl ethers, polyoxyethylene propylene glycol fatty acid esters, and so on.

Suitable pigments include organic and inorganic pigments such as talc, mica, titanium dioxide, titanated mica, iron oxides, ultramarines, chromium oxides, carmine, D&C and FD&C colors and lakes, ferric and ferrous oxides, and so on.

Suitable preservatives include imidazolidinyl urea, the parabens, quaternium 15, benzyl alcohol, phenoxyethanol and so on.

Suitable waxes include beeswax, carnauba, ceresin, microcrystalline, lanolin, paraffin, ozokerite, lanolin alcohol, acetylated lanolin, candelilla, cetyl alcohol, cocoa butter, petrolatum, hydrogenated castor oil spermaceti, bran wax, capok wax, bayberry, etc.

The invention is also directed to a method for moisturizing and forming a film on human skin, nails, or hair comprising applying to the surface an effective amount of extensin protein. An effective amount of extensin is about 0.01-30% by weight. The extensin protein may be applied directly to the surface in a solution form, or it may be incorporated into the cosmetic or pharmaceutical compositions mentioned herein. The extensin protein or protein containing composition may be applied to the surface once or twice a day or as necessary. For example, if the extensin protein is incorporated into a facial moisturizer, usually one to two applications of moisturizer per day will provide a beneficial effect. If the extensin proteins are incorporated into shampoos or hair conditioners, usually application once a day or every other day will be sufficient to provide a beneficial effect. When extensins are incorporated into makeups, blushes, or eyeshadows, they provide a treatment effect to the skin when applied once a day or whenever makeup is worn. If incorporated into nail treatment products or nail enamels, consistent usage in a nail care regimen (i.e. once or twice a week) will provide beneficial results.

The invention will be further described in connection with the following examples which are set forth for the purposes of illustration only. Trade names and registered trademarks are acknowledged as such.

### Example 1

Bean seeds, (Phaseolus Vulgaris) or carrots (Daucus carota) are purchased from local gardening stores and the seeds germinated on mast filter paper in the dark. Germinated seeds are then planted in large window boxes or in gardens. Young plants are grown and then the hypocotyl and roots harvested and homogenized in 50 mM potassium phosphate buffer pH 6,1 mM dithiothreitol (DTT), 0.1 mM phenyl methylsulfonyl fluoride and 2 mM ascorbate in an Osterizer (Waring) blender. The homogenate was diluted with 10 mM calcium chloride and centrifuged at 1000 x G. Pellets were extracted again in 100 mM calcium chloride and recentrifuged. The supernatants were combined and dialyzed to remove excess salt. The result was a crude extensin extract suitable for incorporation into cosmetics or pharmaceuticals.

### Example 2

Small tomato plants are obtained from local gardening stores and acclimated to outdoor gardens. After two weeks small amounts of plant tissue are cut off and frozen in liquid nitrogen until a sufficient amount is collected (approximately 100 grams of wet tissue). Crude homogenates are made and extracted with 75 mM aluminum chloride.

### Example 3

Small pieces of potato are rooted by exposing the pieces to water containing 35 mM sodium azide, 0.5% ferulic acid, 0.05% curcumin, 0.1% carnosine, or 0.05% extract from spinach, gingo bilboa, or ginseng. The root systems formed are harvested and homogenized in 50-100 mM calcium chloride or 150 mM sodium chloride.

### Example 4

Pea seeds (Pisum sativum) are germinated and the epicotyl and root sections were dissected and extracted in a loosely stoppered flask at 70° C. with 0.12% v/v acetic acid and 0.3% sodium chlorite for 30 minutes under a nitrogen blanket. After cooling the solution was filtered through coarse glass wool and the residue washed five times with distilled water. Nitrogen is bubbled through the filtrate to remove any extraneous chlorine dioxide. The resulting homogenate is dialyzed to yield a crude extensin protein preparation.

### Example 5

An extensin containing oil in water moisturizing lotion is made as follows:

| | w/w% |
|---|---|
| Glyceryl stearate | 3.0 |
| PPG-10 lanolin ether | 0.5 |
| Mineral oil | 6.3 |
| Lanolin alcohol | 0.7 |
| Oleic acid | 2.7 |
| Isocetyl stearate | 10.0 |
| Triethanolamine | 1.3 |
| Carbomer 941 | 0.1 |
| Glycerin | 4.0 |
| Preservative | 0.4 |
| Extensin solution from carrots¹ | 5.0 |
| Hydrolyzed extensin polypeptdes² | 5.0 |
| Water | qs 100.00 |

| | |
|---|---|
| ¹ 5% solution | |
| ² 5% peptide solids, "Vegagen", Cenferchem, Inc., Stamford, CT | |

### Example 6

An oil in water moisturizing cream was made as follows:

| | w/w% |
|---|---|
| Glyceryl stearate | 5.0 |
| Cetyl alcohol | 2.0 |
| Stearyl alcohol | 2.0 |
| Isopropyl stearate | 4.0 |
| Mineral oil | 12.0 |
| Polysorbate 60 | 1.0 |
| Glycerin | 8.0 |
| Xanthan gum | 0.25 |
| Preservative | 0.6 |
| Extensin solution from carrots¹ | 5.0 |
| Hydrolyzed extensin polypeptides from carrots² | 5.0 |
| Water | qs 100.00 |

| | |
|---|---|
| ¹ 5% solution | |
| ² Vegegen, Centerchem, Inc, Stamford, CT | |

### Example 7

An oil/water cream makeup was made as follows:

| | w/w% |
|---|---|
| Octyldodecyl stearyl stearate | 4.0 |
| Isocetylstearate | 1.0 |
| Glyceryl stearate | 6.0 |
| Isostearic acid | 2.0 |
| Stearic acid | 1.0 |
| Ceteth 10 | 1.0 |
| Cyclomethicone | 12.0 |
| Stearyl alcohol | 1.5 |
| Nonionic surfactant mixture¹ | 1.0 |
| Binders and thickeners | 1.7 |
| Titanium dioxide | 8.0 |
| Iron oxide | 2.0 |
| Propylene glycol | 3.0 |
| Triethanolamine | 1.5 |
| Preservatives | 0.55 |
| Extensin solution from tomato² | 1.0 |
| Water | qs 100.00 |

| | |
|---|---|
| ¹ lecithin, polysorbate 20, sorbitanlaurate | |
| ² 1.5% solution | |

### Example 9

A water/oil pigment emulsion makeup was made as follows:

| | w/w% | |
|---|---|---|
| Cyclomethicone | 22.6 | 12.0 |
| Dimethicone | ---- | 5.0 |
| Surfactant | 16.0 | 20.0 |
| Laureth 7 | 0.5 | ---- |
| Laureth 9 | ---- | ---- |
| Bentone gellant | 5.0 | ---- |
| Iron oxides | 3.1 | 2.4 |
| Titanium dioxide | 12.0 | 8.5 |
| Talc | 4.5 | 3.3 |
| Sodium chloride | 2.0 | 2.0 |
| Propylene glycol | 6.0 | 8.0 |
| Preservative | 0.5 | 0.5 |
| Extensin solution from potato | 0.5 | 0.5 |
| Water | | qs 100.0 |

### Example 10

A water/oil pigment emulsion eyeshadow was made as follows:

| | w/w% |
|---|---|
| Cyclomethicone | 2.0 |
| Dimethicone | 5.0 |
| Surfactant | 20.0 |
| Peg-7 C₁₂₋₁₅ ether | 0.5 |
| Chromium oxide | 6.2 |
| Ultramarine blue | 4.0 |
| Titanium dioxide coated mica | 6.0 |
| Sodium chloride | 2.0 |
| Propylene glycol | 8.0 |
| Preservatives | 0.3 |
| Extensin solution from potato¹ | 0.5 |
| Water | qs 100.0 |

| | |
|---|---|
| ¹ 1% solution | |

Example 11 A protein shampoo was made as follows:

| | w/w% |
|---|---|
| Ammonium lauryl sulfate | 10.0 |
| Cocamide diethanolamine | 4.0 |
| Cocamidopropyl betaine | 4.0 |
| Ammonium chloride | 0.8 |
| Citric acid | 0.1 |
| Extensin solution from corn (1%) | 1.0 |
| water | qs 100.0 |

### Example 12

A creme rinse hair conditioner was made as follows:

| | w/w% |
|---|---|
| Stearalkonium chloride | 2.0 |
| Cetyl alcohol | 1.0 |
| Stearyl alcohol | 0.5 |
| Ceteareth 20 | 2.0 |
| Xanthan gum | 0.5 |
| Citric acid | 0.3 |
| Dimethicone | 0.2 |
| Extensin solution from corn (1%) | 0.2 |
| Water | qs 100.0 |

### Example 13

A variety of extensin containing personal care products are made as follows:

| | A | B | C | D |
|---|---|---|---|---|
| | w/w% | | | |
| Mineral oil | 20.0 | ---- | ---- | ---- |
| Beeswax | 2.0 | ---- | ---- | ---- |
| Polysorbate 40 | 8.0 | ---- | ---- | ---- |
| Polysorbate 20 | ---- | ---- | 1.0 | ---- |
| PEG 20 sorbitan beeswax | 2.0 | ---- | ---- | ---- |
| Stearic add | 10.0 | ---- | ---- | ---- |
| Petrolatum | 4.0 | ---- | ---- | ---- |
| Sorbitol | 5.0 | ---- | ---- | ---- |
| Ethyl alcohol | ---- | 50.0 | 50.0 | 50.0 |
| Menthol | ---- | 0.05 | ---- | 0.1 |
| Carbomer 940 | ---- | 0.75 | ---- | ---- |
| Triethanolamine | ---- | 0.75 | ---- | ---- |
| Polysorbate 80/acetyllated lanolin alcohol/cetyl acetate | ---- | 3.0 | ---- | ---- |
| Citric acid | ---- | ---- | ---- | 2.0 |
| Glycerol | ---- | ---- | ---- | 2.5 |
| Perfume | qs | 8.0 | 8.0 | 0.5 |
| Preservative | qs | ---- | ---- | ---- |
| Extensin solution from sycamore | 0.5 | 0.1 | 0.1 | 0.1 |
| Water (qs 100) | | | | |

A is a cleansing cream, B is a dear fragrance, C is a toner, D is an after shave.

### Example 14

Two nail enamel preparations were made as follows:

| | w/w% | |
|---|---|---|
| | A | B |
| Beeswax | 12.0 | ------ |
| Lanolin, anhydrous | 15.0 | ------ |
| Cocoa butter | 8.0 | ------ |
| Cetyl alcohol | 3.0 | ------ |
| Chdesterol | 1.0 | ------ |
| Mineral oil | 30.0 | ------ |
| Extensin solution from carrots (0.2%) | 5.0 | 0.5 |
| Preservative | qs | ----- |
| Perfume | qs | ----- |
| Water | qs100 | ----- |
| Nitrocellulose | ----- | 15.00 |
| Toluene sulfonamide | ----- | 7.5 |
| Dibutyl phthalate | ----- | 3.75 |
| Butyl acetate | ----- | 29.35 |
| Ethyl alcohol | ----- | 6.4 |
| Butyl alcohol | ----- | 1.1 |
| Toluene | ----- | 36.40 |

## Claims

1. A composition for cosmetic and/or pharmaceutical use on skin, nails or hair, in which the cosmetically- and/or pharmaceutically-active ingredient is or includes an effective film-forming concentration of a soluble precursor-form extensin protein with or without extensin hydrolysate(s) in which the soluble precursor-form extensin protein is intact extensive(s) having molecular weight(s) of at least 100,000 daltons.

2. A composition as claimed in claim 1, in which the molecular weight(s) of the extensin hydrolysate(s) if present are in the range of from 100 to 1500 dallons.

3. A composition as claimed in claim 1 or claim 2, in which the sole extensin-type active ingredient is the soluble precursor-form extensin protein.

4. A composition as claimed in any of the preceding claims, in which the molecular weight(s) of the intact extensin(s) lie in the range of from 100.000 to 150.000 daltons.

5. A composition as claimed in any of the preceding claims, in which the concentration of the soluble precursor-form extensin protein is in the range of from 0.01 to 30 % by weight.

6. A composition as claimed in any of the preceding claims, in which the concentration of the soluble precursor-form extensin protein is in the range of from 0.1 to 10% by weight.

7. A composition as claimed in any of the preceding claims, in which the concentration of the soluble precursor-form extensin protein is in the range of from 0.5 to 5% by weight.

8. A cosmetic composition as claimed in any of the preceding claims, in which the remainder of the composition is formulated as a cream, lotion, gel. toner, fragrance splash, makeup, blush, eyeshadow, nail-care preparation, aftershave, cleanser, shampoo or hair-conditioner.

9. A pharmaceutical composition as claimed in any of claims 1 to 7, in which the remainder of the composition is formulated as a lotion, cream, ointment, gel or solution.

10. Use of soluble precursor-form extensin protein, with or without extensin hydrolysate(s), in the manufacture of a medicament for the therapeutic treatment of skin, nails or hair in which the soluble precursor-form extensin protein is intact extensin(s) having molecular weight(s) of at least 100,000 daltons.

## Patentansprüche

1. Mittel zur kosmetischen und/oder pharmazeutischen Verwendung auf der Haut, den Nägeln oder dem Haar, in dem der kosmetisch und/oder pharmazeutisch wirksame Bestandteil ein lösliches Vorläuferform-Extensinprotein mit oder ohne Extensinhydrolysat(en) in einer zur Filmbildung wirksamen Konzentration enthält oder daraus besteht, wobei das lösliche Vorläuferform-Extensinprotein intaktes Extensin (intakte Extensine) mit einem Molekulargewicht von mindestens 100.000 Dalton ist.

2. Mittel nach Anspruch 1, in dem das Molekulargewicht des Extensinhydrolysats (der Extensinhydrolysate), wenn vorhanden, im Bereich von 100 bis 1.500 Dalton liegt.

3. Mittel nach Anspruch 1 oder 2, in dem der einzige wirksame Bestandteil vom Extensintyp das lösliche Vorläuferform-Extensinprotein ist.

4. Mittel nach einem der vorstehenden Ansprüche, in dem das Molekulargewicht des intakten Extensins (der intakten Extensine) im Bereich von 100.000 bis 150.000 Dalton liegt.

5. Mittel nach einem der vorstehenden Ansprüche, in dem die Konzentration des löslichen Vorläuferform-Extensinproteins im Bereich von 0,01 bis 30 Gew.% liegt.

6. Mittel nach einem der vorstehenden Ansprüche, in dem die Konzentration des löslichen Vorläuferform-Extensinproteins im Bereich von 0,1 bis 10 Gew.% liegt.

7. Mittel nach einem der vorstehenden Ansprüche, in dem die Konzentration des löslichen Vorläuferform-Extensinproteins im Bereich von 0,5 bis 5 Gew.% liegt.

8. Kosmetisches Mittel nach einem der vorstehenden Ansprüche, in dem der Rest des Mittels als Creme, Lotion, Gel, Toner, Duftwasser (fragrance splash), Makeup, Rouge, Lidschatten, Nagelpflegemittel, Aftershave, Reiniger, Shampoo oder Haarweichspüler formuliert ist.

9. Arzneimittel nach einem der Ansprüche 1 bis 7, in dem der Rest des Mittels als Lotion, Creme, Salbe, Gel oder Lösung formuliert ist.

10. Verwendung von löslichem Vorläuferform-Extensinprotein mit oder ohne Extensinhydrolysat(en) zur Herstellung eines Arzneimittels zur therapeutischen Behandlung von Haut, Nägeln oder Haar, wobei das lösliche Vorläuferform-Extensinprotein intaktes Extensin (intakte Extensine) mit einem Molekulargewicht von mindestens 100.000 Dalton ist.

## Revendications

1. Composition cosmétique et/ou pharmaceutique destinée à être utilisée sur la peau, les ongles ou les cheveux, dans laquelle le principe actif du point de vue cosmétique et/ou pharmaceutique, est ou contient une concentration efficace formant pellicule d'une protéine soluble sous forme de précurseur de l'extensine, avec ou sans hydrolysat(s) d'extensine, dans laquelle la protéine soluble sous forme de précuseur de l'extensive est une (des) extensine(s) intacte(s) ayant un (des) poids moléculaire(s) d'au moins 100,000 daltons.

2. Composition selon la revendication 1, dans laquelle le (les) poids moléculaire(s) de l'(des) hydrolysat(s) d'extensine, si présent(s), se situe(nt) dans l'intervalle de 100 à 1500 daltons.

3. Composition selon la revendication 1 ou la revendication 2, dans laquelle l'unique principe actif de type extensine est la protéine soluble sous forme de précurseur de l'extensive.

4. Composition selon l'une des revendications précédentes, dans laquelle le (les) poids moléculaire(s) de l'(des) extensine(s) intacte(s) se situe(nt) dans l'intervalle de 100,000 à 150,000 daltons.

5. Composition selon l'une des revendications précédentes, dans laquelle la concentration de la protéine soluble sous forme de précurseur de l'extensine se situe dans l'intervalle de 0,01 à 30 % en poids.

6. Composition selon l'une des revendications précédentes, dans laquelle la concentration de la protéine soluble sous forme de précurseur de l'extensine se situe dans l'intervalle de 0,1 à 10 % en poids.

7. Composition selon l'une des revendications précédentes, dans laquelle la concentration de la protéine soluble sous forme de précurseur de l'extensive se situe dans l'intervalle de 0,5 à 5 % en poids.

8. Composition cosmétique selon l'une des revendications précédentes dans laquelle le reste de la composition est formulé sous forme de crème, lotion, gel, lotion tonifiante, aspersion parfumée, maquillage, fard à joues, ombre à paupières, préparation pour le soin des ongles, après-rasage, démaquillant, shampooing ou démélant.

9. Composition selon l'une des revendications 1 à 7, dans laquelle le reste de la composition est formulé sous forme de lotion, crème, pommade, gel ou solution.

10. Utilisation d'une protéine soluble sous forme de précuseur de l'extensine, avec ou sans hydrolysat(s) d'extensine, pour la préparation d'un médicament destiné au traitement théiapeutique de la peau, des ongles ou des cheveux, dans laquelle la protéine soluble sous forme de précuseur de l'extensine est une (des) extensine(s) intacte(s) ayant un (des) poids moléculaire(s) d'au moins 100,000 daltons.
